# EUROPEAN PATENT APPLICATION

(11) **EP 2 116 260 A1**
(43) Date of publication of application: **11.11.2009**
(21) Application number: 08007460.2
(22) Date of filing: 16.04.2008
(51) Int. Cl.: A61K 39/395, C07K 14/47, G01N 33/50

(54) **Screening method for agents suitable for prophylaxis and therapy of Alzheimer's disease (AD)**

(71) Applicant: Freie Universität Berlin, 14195 Berlin (DE)
(72) Inventor: Multhaup, Gerd, Prof.Dr., 14532 Kleinmachnow (DE); Harmeier, Anja, Dr., 10585 Berlin (DE)
(74) Representative: Schüssler, Andrea

(57) **Abstract**

Described is a method of screening for a therapeutic agent useful in the prophylaxis or treatment of AD comprising detecting oligomers of a peptide comprising an APP-G₂₉xxxG₃₃ motif in an assay system to which system a test compound has been added and comparing the size distribution of the oligomers of said peptide and/or degree of oligomerization with a control assay characterized by the absence of said test compound, wherein reduction of the amounts of dimers and/or tetramers indicates that said test compound might be suitable for prophylaxis or treatment of AD.

## Description

The present invention relates to a method of screening for a therapeutic agent capable of eliminating the formation of toxic oligomers (preferably dimers and tetramers) of an Aβ peptide which is useful in the prophylaxis or treatment of AD. These oligomers can also serve as diagnostic markers.

The deposition of amyloid beta (Aβ) in the form of senile plaques in sufficient quantity is a pathological hallmark of Alzheimer's disease (AD). Currently, Aβ is regarded as the most important diagnostic parameter since this peptide is responsible for the plaque-like extracellular depositions (amyloidosis) in the brain which is a characteristic feature in the late course of AD. Aβ exists in varying lengths and is derived from beta- and gamma-secretase cleavage of amyloid precursor molecule (APP). APP is a type 1 transmembrane (TM) protein, which undergoes proteolytic processing by several secretases. First, the bulk of the ectodomain needs to be removed by membrane-bound α- or β-secretases leading to secreted forms of APP and membrane bound C-terminal fragments α-CTF or β-CTF, respectively. Regulated intramembrane proteolysis (RIP) of the β-CTF by γ-secretase occurs only after ectodomain shedding and releases the Aβ peptide from the membrane. The active γ-secretase complex consists of the four subunits presenilin-1 (PS-1), APH-1, PEN-2 and nicastrin. PS-1 contains two catalytic active aspartate residues in TMS-6 and TMS-7. PS-1 is endoproteolytically cleaved and the N-terminal and C-terminal fragments were shown to exist as dimers in the catalytic core of γ-secretase. The amino termini of CTF stubs are recognized by nicastrin, which functions as a γ-secretase substrate receptor. The γ-secretase cleaves at variable sites thus generating Aβ peptides of varying lengths. Aβ peptides are closely linked to AD as they accumulate to oligomers, further to fibrils and subsequently to amyloid plaques. Aβ42 is the pathologically most relevant form of the Aβ species since it is more prone to aggregation forming dimers, tetramers etc. progressively and, finally, amyloid fibrils and is found elevated in AD patient brains.

It could be shown in APP transgenic mice that loss of cognitive performance does not correlate with the formation of amyloid plaques but is due to a damage of synapses and loss of neurons prior to the formation of plaques. In particular, soluble forms of Aβ, the so-called Aβ oligomers, are assumed to be responsible for the loss of neurons and, as a consequence, decrease of cognitive performance - prior to the aggregation of Aβ species to fibrils. However, so far it is not known (a) which forms of Aβ oligomers exert these toxic effects and (b) how the Aβ oligomers cause the AD pathologies.

Recently, a G₂₉xxxG₃₃ motif could be identified mediating helix-helix interactions within the transmembrane segment of APP. This motif represents a unique homophilic interaction site of the γ-secretase substrate CTF and controls the processing into Aβ via the final γ-secretase cleavages. APP TMS dimerization is unique insofar as it is the only known γ-secretase substrate with a GxxxG motif in triplicate at the start of the TMS. In a neuronal cell system, mutations at G₂₉ and G₃₃ of the GxxxG motif gradually attenuate the TMS dimerization strength, specifically reduce the formation of Aβ42, leave the level of Aβ40 unaffected, but increase Aβ38 and shorter Aβ species. In fact, the glycine residues G₂₉ and G₃₃ are part of a dimerization site within the TMS, but do not impair oligomerization of the APP ectodomain. It can be concluded that dimerization of APP TMS is a risk factor for AD due to facilitating Aβ42 production.

Currently, only symptomatic therapies of AD are available. At best, these therapies can slow down progression of the disease, however, there is only a temporarily effect. In other words, currently available therapies (as well as therapies being based on drugs that are currently assayed in clinical trials) cannot stop worsening of the general condition of the patient and, of course, cannot lead to an improvement of the poor health. Currently available drugs are inhibitors of acetylcholine esterases, compounds decreasing the cholesterol level, statines etc.

Thus, the technical problem underlying the present invention is to provide drugs that are useful for the prophylaxis and/or causative therapy Alzheimer's disease (AD).

The solution to said technical problem is achieved by providing the embodiments characterized in the claims.

Starting from the observation that soluble oligomers of Aβ might trigger off AD it was the idea that these oligomers might be target molecules for the development of a therapeutic intervention prior to the onset of the clinical phase allowing decelerating or arresting the disease. In the studies leading to the present invention it was shown that the GxxxG motif not only affects the processing to Aβ with the TMS but also affects aggregation - independent from the TMS and independent from a membrane environment. As long as Aβ is part of APP or β-CTF and embedded in the membrane the sequence is alpha-helical. As soon as Aβ is released from APP the sequence is converted into beta-sheet structure. Thus, aggregation mediated by the GxxxG motif in the membrane affects APP processing (see above) whereas the GxxxG motif in soluble forms of Aβ promote the aggregation of β-sheet structured Aβ molecules. Apparently, Aβ1-42 tetramers show the highest toxicity for human neuroblastoma cells as well as primary hippocampal rat neurons. By use of electrophysiological studies being based on the testing of brain sections of rats for learning aptitude *ex vivo* it could be demonstrated that Aβ1-42 tetramers cause a measurable loss of cognitive performance. This toxic effect and LTP inhibition can be neutralized by replacing the glycine residue at position 33 by an alanine residue or isoleucine residue. The screening method of the present invention allows isolating drugs from a pool of compounds comprising various classes of substances, i.e. drug candidates inhibiting the formation of low MW oligomers, such as dimers and/or tetramers. In this regard the results of the present invention show that in particular wild type tetramers play a key role for inducing loss of neurons, are responsible for cognitive deficiencies and represent a target for new therapeutic interventions. In addition, it was shown that the negative effects can be blocked by a single substitution at position 33 (glycine).

### BRIEF DESCRIPTION OF THE FIGURES

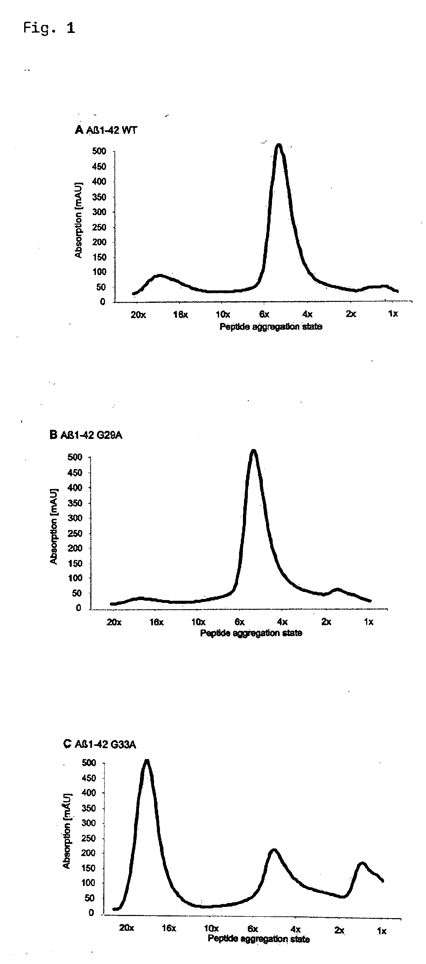

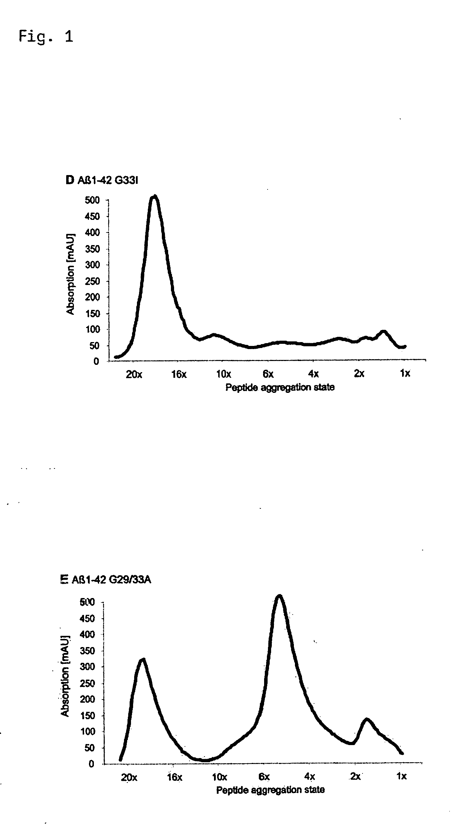

### Figure 1: Size exclusion chromatography of freshly dissolved Aβ1-42 peptides

**(A)** Aβ1-42wt immediately aggregates to 4- and 6-mers and only minor amounts of oligomers with higher MW are formed.
**(B)** Similar effects are observed with Aβ1-42G29A peptides.
**(C)** However, the peptide Aβ1-42G33A shows a shift to oligomers with a higher MW and only minor amounts of small oligomers are detectable.
**(D)** With the peptide Aβ1-42G33I this shift is even expanded.
**(E)** By contrast, the double mutant G29/33A exhibits a balance: Most of the oligomers have a low MW, but large amounts of oligomers having a higher MW are already visible.

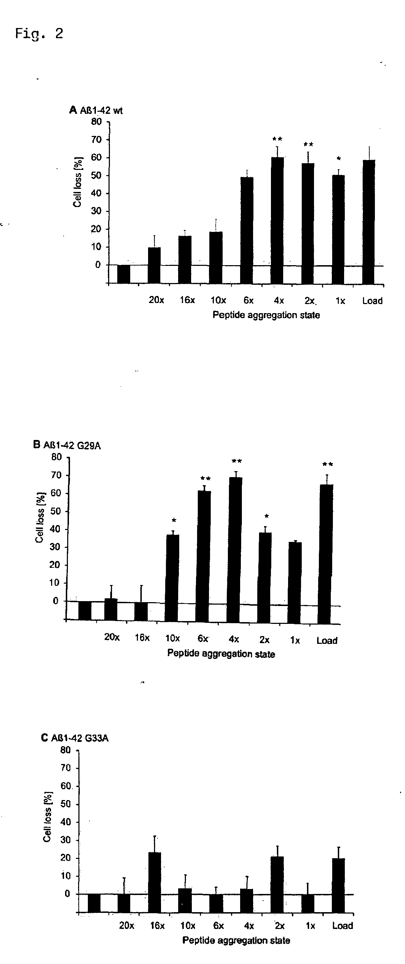

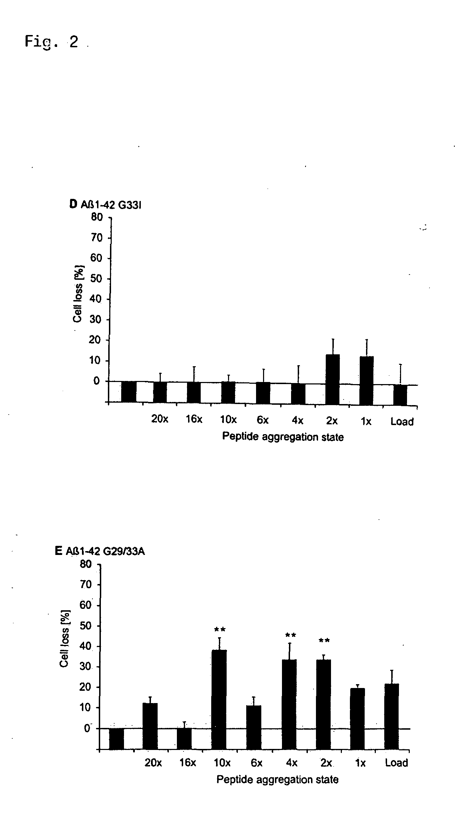

### Figure 2: Toxicity test (MTT) with human neuroblastoma cells (SH-SY5Y)

When applying a standardized concentration of peptides it becomes apparent that Aβ1-42wt **(A)** as well as Aβ1-42G29A **(B)** exhibit a strong toxic effect which is most pronounced for the tetramers. The size of the oligomers inversely correlates with toxicity. Both G33 mutants **(C + D)** do not exhibit significant toxicity. Aβ1-42G29/33A shows only weak toxicity **(E).**

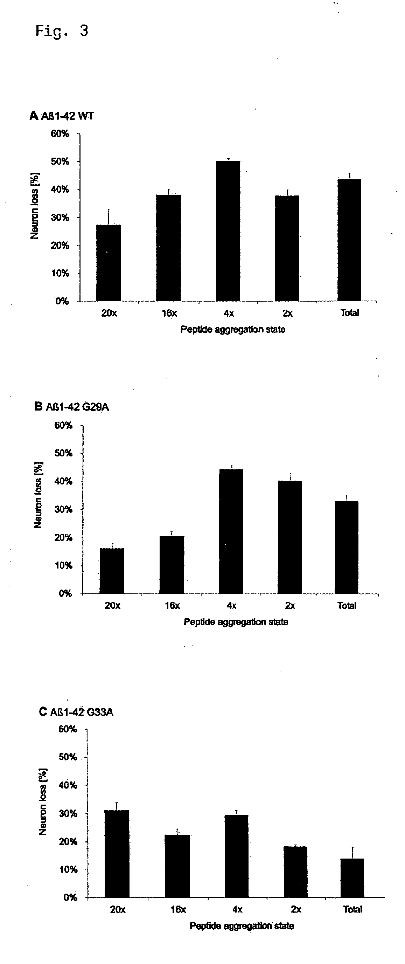

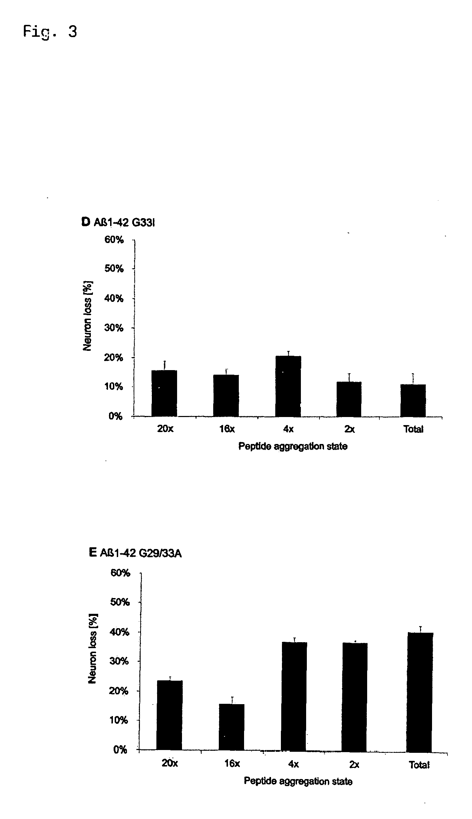

### Figure 3: Toxicity test (Multi-tox) with primary hippocampal rat neurons

It becomes again apparent that when applying a normalized concentration of peptides Aβ1-42wt **(A)** as well as Aβ1-42G29A **(B)** both kinds of peptides exhibit a strong toxic effect which is most pronounced for the tetramers. The degree of oligomerization inversely correlates with toxicity. Both, G33 mutants **(C + D)** result in drastically reduced toxicity. Aβ1-42G29/33A shows weak toxicity **(E).**

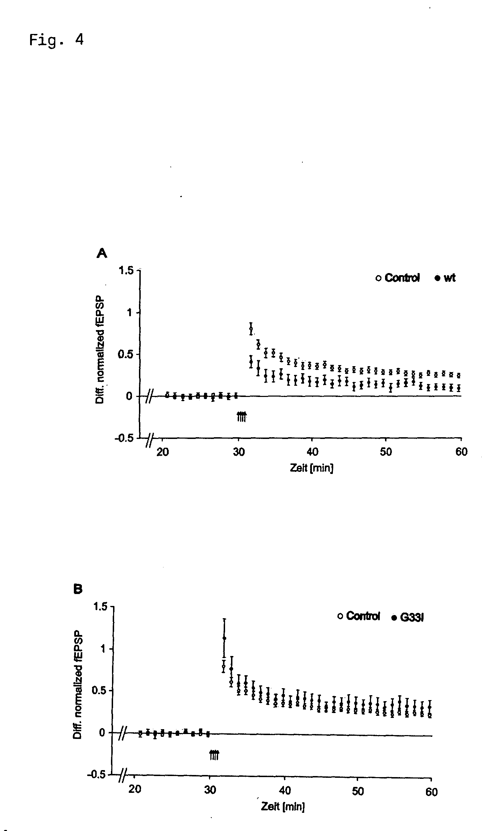

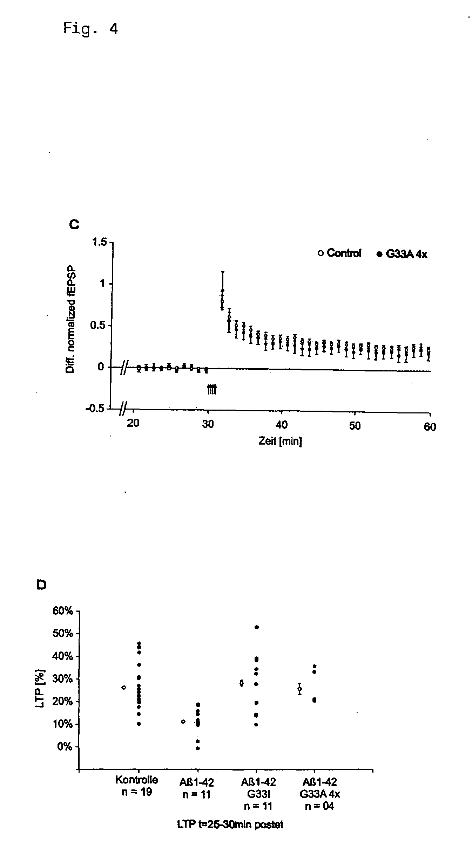

### Figure 4: Long term potentiation (LTP) of acute hippocampal rat brain sections or slices

**(A)** LTP induction was performed with a physiological theta burst 20 min. after incubation with 500 nM Aβ1-42wt peptide. As already known, no LTP is induced in comparison with the control.
**(B)** However, after incubation of the sections with Aβ1-42G33I peptide the induced LTP shows the same value as the control.
**(C)** Results of incubation with Aβ1-42G33A tetramers correspond to the results obtained for Aβ1-42G33I.
**(D)** The evaluation by percentage and comparison of potentiation are shown. No significant differences between the control and the Aβ1-42G33 peptides can be observed (LTP is not inhibited).

The present invention, thus, provides a method of screening for a therapeutic agent useful in the prophylaxis or treatment of Alzheimer's Disease (AD) comprising the steps of
(a) detecting oligomers of an Aβ peptide containing a G₂₉xxxG₃₃ motif in an assay system under conditions allowing oligomerization of said peptide and to which system a test compound has been added; and
(b) comparing the size distribution of the oligomers of said peptide and/or degree of oligomerization of Aβ with a control assay characterized by the absence of said test compound
wherein reduction of the amounts of the toxic Aβ oligomers, preferably Aβ dimers and/or tetramers, indicates that said test compound might be suitable for the prophylaxis or treatment of AD.

The term "oligomer" as used herein means any multimer of the Aβ G₂₉xxxG₃₃ motif containing peptide starting from dimers to higher oligomeric forms. These have a low molecular weight in the range of 8-80 kDa. These may be even-numbered (e.g. dimers or tetramers) and un-even-numbered multimers (e.g. trimers), with even-numbered multimers being preferred.

The term "reduction of the amounts of toxic oligomers, preferably dimers and/or tetramers" as used herein indicates that the test compound is capable of inhibiting the formation of such oligomers and/or neutralizing or de-oligomerizing such oligomers. "De-oligomerizing" means in its most preferred sense "monomerizing". Monomers are no longer toxic and can be degraded, e.g. by IDE. But also compounds for increasing aggregation can be analysed and may be useful as AD therapeutics since higher oligomers are not toxic and the plaque deposits are not the major cause for the plaque deposition.

The test compounds may be very different compounds, both naturally occurring compounds and synthetic, organic and inorganic compounds as well as polymers (e.g. oligopeptides, polypeptides, oligonucleotides and polynucleotides) as well as small molecules, antibodies, sugar, fatty acids, nucleotides and nucleotide analogs, analogs of naturally occurring structures (e.g. peptide "imitators", nucleic acid analogs, etc.) and numerous other compounds. These test compounds should be suitable to inhibit aggregation of membrane free soluble Aβ oligomers inheriting a β-sheet structure and thereby reducing neuronal loss.

A large number of possibly useful test compounds can be screened in extracts of natural products as a starting material. Such extracts may be derived from a large number of sources, e.g. the following species: fungi, actinomycetes, algae, insects, protozoa, plants and bacteria. The extracts showing activity can then be analyzed for isolating the active molecule.

In a preferred embodiment of the screening method of the present invention, the test compound is from a library of fungal extracts. A particularly preferred library of fungal extracts is from a *Penicillium* or *Aspergillus* species.

The test compound may be a single compound or a plurality of compounds. Thus, test compounds can also be screened for on a large scale, e.g. by screening a very large number being able to contain synthetic or natural molecules. Thus, the test compound of a preferred embodiment of the method according to the invention forms part of a substance library. The term "plurality of compounds" in the screening method of the invention is to be understood as a plurality of substances which may or may not be identical. The plurality of compounds may be, e.g., added to the reaction mixture, culture medium, injected into a cell, preferably a mammalian cell, or otherwise applied to a non-human transgenic animal.

If a sample containing a compound or a plurality of compounds is identified in the method of the invention, then it is either possible to isolate the compound from the original sample identified as containing the compound capable of suppressing oligomerization or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the screening method of the invention only comprises a limited number of substances or only one substance. Preferably, said sample comprises substances of similar chemical and/or physical properties, and most preferably said substances are identical.

Fundamentally suited assay formats for identifying positive test compounds are well known in the biotechnological and pharmaceutical industries and additional assays and variations of the above assays provided for the purpose of illustration are obvious to the person skilled in the art. The person skilled in the art can, e.g., draw on the assay formats given in the examples, below.

Compounds isolated by the screening method of the invention are suitable as therapeutics to treat, prevent, control, or lessen the severity of AD, or can be used as lead compounds for the development of analogue compounds, i.e. drugs that have additional desired properties, e.g. are capable of efficiently passing the blood-brain barrier. Moreover, such drugs should have a stabilized electronic configuration and molecular conformation that allows key functional groups to be presented in substantially the same way as the lead compound. In particular, the analog compounds have spatial electronic properties which are comparable to the binding region, but can be smaller molecules than the lead compound, frequently having a molecular weight below about 2 kD and preferably below about 1 kD. Identification of analog compounds can be performed through use of techniques such as self-consistent field (SCF) analysis, configuration interaction (CI) analysis, and normal mode dynamics analysis. Computer programs for implementing these techniques are available; e.g., Rein, Computer-Assisted Modeling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said analogues can be tested for their effects according to methods known in the art; see also supra. Furthermore, peptidomimetics and/or computer aided design of appropriate derivatives and analogues can be used according to routine methods.

For certain purposes, e.g. for determination of the degree of oligomerization, the peptide containing the APP-G₂₉xxxG₃₃ motif can be detectably labeled, for example, with a radioisotope, a bioluminescent compound, a chemiluminescent compound, a fluorescent compound, a metal chelate, or an enzyme.

The APP-G₂₉xxxG₃₃ motif containing peptide can also be present as a fusion protein provided that the presence of the fusion partner does not interfere with oligomerization. The fusion partner, i.e., second polypeptide, can be a polypeptide being capable of generating a detectable signal. The second polypeptide segment of a fusion protein comprising a reporter protein can be a full-length protein or a protein fragment. Proteins commonly used in fusion protein construction include, but are not limited to beta-galactosidase, beta-glucuronidase, green fluorescent protein (GFP), YFP, autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions.

In a more preferred embodiment of the screening method of the present invention, the critical oligomers are tetramers, i.e. multimers which exhibit the highest toxicity.

The screening method of the present invention can be carried out in any assay system allowing (a) oligomerization of the peptide and (b) interaction of the test compound with the peptide. In a preferred embodiment step (a) may be carried out under physiological conditions although it has been observed that the peptide forms oligomers also under unphysiological pH conditions. The oligomerization of the Aβ peptide is an unavoidable process that occurs automatically. This aggregation may lead after some time to the formation of Aβ fibrils may be avoided to some extent by using a low temperature (e.g. about 4°C) and addition of DMSO.

For example, the assay system can be an *in vitro* system, e.g., a cell-free extract or cell lysate, an *in vivo* system using suitable cell lines (cell based system) or a non-human animal. The test compound(s) can be added to the cell-free extract, cell lysate or a cell culture at various concentrations. Suitable cells are known to the person skilled in the art and easily available. Examples of suitable cells are higher eucaryotic cells, e.g., HEK293 etc. In further preferred embodiments, the cells are continuous mammalian cell lines including, without limitation, human, monkey or murine cells, preferably neuronal cells, e.g., SH-SY5Y (human neuroblastoma cells), N2a (primary mouse neuroblastoma cells). Preferably, the cells include exogenous an APP-G₂₉xxxG₃₃ motif containing peptide (e.g., APP-CTF, APP or APPsw) encoding DNA (i.e., DNA artificially introduced into the cells, e.g., by transfection, infection, transformation etc. according to routine methods) effecting expression of the gene, preferably over expression.

In a further preferred embodiment of the screening method of the present invention, the assay system is a non-human animal, preferably a transgenic mouse or rat. Preferably, said non-human animal is transformed to express a gene encoding wildtype or variants of the APP-G₂₉xxxG₃₃ motif containing peptide. These transgenic animals expressing the variants may be used as controls in the assay system since these "variants" show differences with regard to aggregation behaviour and are less toxic than the "wildtype". In particular, the "Ala" variants are less toxic and aggregate to higher forms and may be useful to find compounds suitable for causing de-aggregation.

Methods for the generation of transgenic animals are extensively described in the literature and, thus, known to the person skilled in the art; see, e.g., US Patent Number 5,720,936. For example, transgenic mice with the human APP promoter linked to *E. coli* β-galactosidase (Wirak et al., EMBO J. 10, 289-296 (1991)) as well as transgenic mice expressing the human APP751 cDNA (Quon et al., Nature 352, 239-241 (1991)) or subfragment of the cDNA induding the β peptide (Wirak et al., Science 253, 323-325 (1991); Sandhu et al., J. Biol. Chem. 266, 21331-21334 (1991); Kawabata, Nature 354, 476-478 (1991)) have been produced. For example, the peptide encoding gene constructs can be prepared using the naturally occurring APP promoter of human, mouse, or rat origin, as well as inducible promoters such as the mouse metallothionine promoter that can be regulated by addition of heavy metals, such as zinc to the animal's water or diet.

Neuron-specific expression of constructs can be achieved by using the rat specific enolase promoter. Examples of further promoters that are suitable for the purposes of the present invention include the β-actin gene promoter, human platelet derived growth factor B (PDGF-B) chain gene promoter, rat sodium channel gene promoter, mouse myelin basic protein gene promoter, human copper-zinc superoxide dismutase gene promoter, and mammalian POU-domain regulatory gene promoter.

The constructs controlled by these promoters are introduced into animal embryos using standard techniques such as microinjection or embryonic stem cells. Cell culture based models can also be prepared by two methods. Cell cultures can be isolated from the transgenic animals or prepared from established cell cultures using the same constructs with standard cell transfection techniques.

Preferably, the peptide used in the screening method of the present invention is a form of Aβ that covers a multiple, preferably triplicate, GxxxG motif or Aβ1-42 or a fragment thereof containing the G₂₉xxxG₃₃ motif. This peptide can be mutated and, preferably, the G₃₃ of said motif is mutated. Particularly preferred is a mutation characterized by the replacement of G by A or I at position 33. With these structurally different mutants it is possible to identify test substances which bind to non-toxic oligomers but not to those which are toxic. Thus, it would be possible to determine whether and when toxic forms exist and to which test substances they bind and vice versa.

Numerous well known methods are available for determining the degree of oligomerization and/or the size distribution of the oligomers. In a preferred embodiment of the screening method of the invention the size distribution of the oligomers and/or the degree of oligomerization is determined by the use of a binding substance the binding affinity of which depends on the degree of oligomerization, e.g. detection and/or quantification of oligomers is accomplished by immunological methods using specific antibodies or fragments thereof. The term "antibody" as used herein, preferably, relates to antibodies which consist essentially of pooled monoclonal antibodies with different epitopic specificities, as well as distinct monoclonal antibody preparations. Monoclonal antibodies are made from an antigen containing fragments of the Aβ peptides by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody.

The antibodies can be detectably labelled, for example, with a radioisotope, a bioluminescent compound, a chemiluminescent compound, a fluorescent compound, a metal chelate, or an enzyme (e.g. horse-radish peroxidase, alkaline phosphatase, beta-galactosidase, malate dehydrogenase, glucose oxidase, urease, catalase etc.) which, in turn, when later exposed to a substrate will react to the substrate in such a manner as to produce a chemical moiety which can be detected. The antibodies can also be immobilized on an insoluble carrier, e.g. glass, polystyrene, polypropylene, polyethylene, dextran, nylon, natural and modified celluloses, polyacrylamides, agarose and magnetic beads.

Any of a wide range of well known immunodetection techniques can be used. Examples of immunoassays suitable for the method of the present invention are competitive or sandwich assays, such as the enzyme linked immunosorbent assay (ELISA), the radioimmunoassay (RIA) or Western Blots. Suitable antibody assay labels are known in the art and include enzyme labels, such as, glucose oxidase, and radioisotopes, such as iodine (¹²⁵I, ¹²¹I), carbon (¹⁴C) sulphur (³⁵S), tritium (³H), indium (¹¹²In), and technetium (⁹⁹mTc), and fluorescent labels, such as fluorescein and rhodamine.

Preferred techniques are ELISA and immunoprecipitation. Detection and/or quantification can also be carried out by MALDI or ESI-MS analysis.

Alternatively, the size distribution of the oligomers and/or the degree of oligomerization is defined by the determination of the MW using methods well-known to the person skilled in the art, e.g., SEC (Size Exclusion Chromatography) or Western Blots(using an antibody as described above). In this regard it should be considered that SDS gels show different separation properties for the oligomers than native separation gels since SDS causes that protofibrills are divided into smaller oligomers.

In case of an assay format employing a non-human animal, preferably a transgenic mouse, oligomerization, in particular the inhibition of formation of dimers and/or tetramers can also be screened via the determination of toxicity, e.g., by use of the tests as described in the examples, below, or by monitoring behavioural alterations employing behavioural tests designed to assess learning and memory deficits. An example of such as test is the Morris Water maze test (Morris, Learn Motivat 12:239-260 (1981)). In this procedure, the animal (e.g., transgenic mouse or rat) is placed in a circular pool filled with water, with an escape platform submerged just below the surface of the water. A visible marker is placed on the platform so that the animal can find it by navigating toward a proximal visual cue. Alternatively, a more complex form of the test in which there are no formal cues to mark the platform's location will be given to the animal. In this form, the animal must learn the platform's location relative to distal visual cues.

The assay involving non-human transgenic animals as described above can be employed for an initial screening or for confirming and/or refining results obtained with an *in vitro* assay or cell based assay of the present invention.

It is also possible to carry out the assay in living cells and tissues (e.g. brain sections) in a "real-time" format, e.g. by FRET. Based on this it is possible to establish an imaging method (e.g. MRT) using compounds that bind to the oligomers.

Then it may be possible to determine in patients when the oligomers have reached a critical concentration and mass and neuronal loss takes place.

The present invention also provides an antibody specifically binding to a dimer and/or tetramer of a peptide comprising an APP-G₂₉xxxG₃₃ motif for vaccination (a) of a subject suffering from AD or (b) for limiting or eliminating a risk to contract AD. Suitable antibodies and methods for generating such antibodies are described above. In this regard it has been discovered to be advantageous to chemically stabilize the antigen molecules (e.g. via S-S- bridges, cross-linking, etc.). Another approach is to use compounds like inositol (McLaurin et al. J.Mol.Biol. 1998; McLaurin et al. JBC 2000; McLaurin et al. J.Mol.Med 2007) or inhibitor peptide (Liu et al., Biochemistry 2005) to inhibit formation of fibrils and to keep the antigen in oligomeric structure. Furthermore, it is possible to stabilize the oligomers by using a certain preparation technique (Dahlgren et al. JBC 2002; Stine et al. JBC 2003). These different measures make sure that the peptide is kept in oligomeric (preferably dimeric or tetrameric) structure and can be used as antigen for vaccination after the appropriate separation and purifying steps (e.g. via SEC). Such a vaccine can be used to stimulate humoral and/or cellular immune response in subjects who may benefit from such responses.

Finally, the present invention relates to
(a) a dimer or tetramer of a peptide comprising an APP-G₂₉xxxG₃₃ motif, wherein, in a preferred embodiment, said peptide is Aβ40 or Aβ42 or a fragment thereof containing the APP-G₂₉xxxG₃₃ motif; and
(b) a peptide comprising an APP-G₂₉xxxG₃₃ motif wherein the G₃₃ of the APP-G₂₉xxxG₃₃ motif is mutated. Preferably, G₃₃ has been replaced by A₃₃ or I₃₃.

The following examples illustrate the invention.

### Example 1

### General Methods

### Peptide synthesis/Preparation of Peptides

Peptides were synthesized at automated synthesizer (ABI 433a, Applied Biosystem, Forster City, CA) on Trt-Tentagel resin (Rapp-Polymere (Tübingen, Germany, 0.25 mmol/g, 0.5 g) using N-(9-fluorenyl)methoxycarbonyl(Fmoc)-strategy (double couplings with 9 equi. of Fmoc-aa/ HBTU/ 6 equi. DIPEA). After final cleavage/ deprotection using trifluoroacetic acid/H₂O (9/1), crude peptides were purified by preparative RP-HPLC to give final products of 95% purity according to HPLC analysis (detector 220 nm). For cyclisation (disulfide formation) linear peptide (1 mg/mL) was dissolved in sodium bicarbonate buffer at pH 8.5 and the mixture was stirred exposed to air for 3 days. After lyophilisation the crude product was purified by preparative HPLC and characterised by mass MALDI spectrometry, which gave the expected masses for the linear and cyclic peptides.

For review purposes reference is made to M. Beyermann, M., Fechner, K., Furkert, J., Krause, E., and Bienert, M. (1996) J. Med. Chem. 39, 3324-3330

### Size exclusion chromatography:

In order to discriminate the various oligomeric forms of Aβ wildtype and mutant peptides, a size exclusion chromatography was performed. Therefore, a Superose12 column (Amersham Bioscience) was equilibrated with 1x PBS (137 mM NaCl, 2,7 mM KCl, 10 mM Na2HPO4, 2 mM KH2PO4) under conditions recommended by the manufacturer. Thereafter 1 mg peptide was monomerized with 50 µl concentrated formic acid and evaporated. Peptides were solubilized in H₂O containing 0,125% NH₃ to a final concentration of 1 mg/ml.

A volume of 500 µl was centrifuged at maximal speed for 10 minutes and loaded on the column with a flow rate of 0.5 ml/min. For determination of the peptide concentration, fractions were tested with Bradford-, BCA- assay and dot blot analysis. The collected fractions were immediately applied for further analysis.

### MTT Assay:

SH-SY5Y cells were routinely cultured in DMEM/Ham'sF12 medium (PAA) supplemented with 10% (v/v) bovine calf serum, 2 mM glutamine, 0,1 mM non-essential amino acids and 0,5 mM sodium pyruvate in a 5% CO₂ humidified atmosphere at 37°C.

Cells were plated at a density of 1.000 cells per well in 100 µl of fresh medium on 96- well plates. After 48 h medium was replaced by DMEM/Ham's F12 with 100U ml⁻¹ penicillin and 10 µg streptomycin including 10 µM peptide, freshly dissolved in H₂O containing 0,125% NH₃ or fractions received by size exclusion chromatography. Probe and control were incubated for 12 h. Cell mediated reduction of 3-(4,5-dimethylthiazol-2-yl)-2,5-diphenyltetrazolium bromide (MTT) was used to monitor Aβ toxicity by addition of 10 µl of a 5 mg ml⁻¹ MTT stock solution and incubation for further 1-2 h. Before measuring the absorbance of blue formazan at 562nm using an automatic micro plate reader, cells were lysed with 50 µl DMSO for 10 minutes at room temperature.

### Toxicity of AB1-42 on primary hippocampal neurons:

Hippocampi from P0-P1 Wistar rat pups were used for primary neuronal dissociated cultures. Tissue was dissected in HBSS containing Ca²⁺ and Mg²⁺ and incubated with an enzyme solution containing 1 mM CaCl₂, 0.5 mM EDTA pH 8.0, 2 mg/ml Cystein and 200-250 units papain (Worthington) in BME for 60min at 37°C. Supernatant was removed and cells were incubated in inactivation solution (2.5 mg/ml BSA, 2.5 mg/ml Trypsin inhibitor (both Sigma) and 10% FCS in BME) for 5 min at. RT. Tissue clumps were dissociated by repeated passage through 1 ml pipettes. Cells were centrifuged for 10 min at 12000 rpm, 4 °C and resuspended in Neurobasal A medium supplemented with 2% B27, 2 mM Glutamax and 0.2% Penicillin/ Streptomycin (all Gibco). Neurons were cultured on glia cell feeder layers in 96well plates with 5x10³ cells in 100µl medium. For feeder layer cultures rat P0- P1 cortices were prepared as described above and cultured at a density of 1x10⁶ cells in a T75 flask for at least one week in BME medium supplemented with 10% FCS, 5 mM glucose, 1 mM Glutamax, 10mM Hepes, 2.5 µg/ml insulin, 0.2% Penicillin/ Streptomycin. Cells were trypsinized and 2x10³ cells per well were seeded in 96 wells plates four days before neurons were added.

After ten days of incubation neuronal cultures were treated with Aβ1-42 freshly dissolved in H₂O or with SEC fractions for another 48 h. Neuronal viability was detected by using MultiTox-Fluor Multiplex Cytotxicity assay (Promega) as recommended by the producer. Cells treated with buffer only were set as non-toxic.

### LTP measurement:

Despite analysing the oligomerization and toxicity of Aβ wildtype and mutant peptides, electrophysiological measurements were performed.

Hippocampal slices were prepared from 3- 6 week old Wistar rats. Rats were decapitated under ether anesthesia. The brain was removed quickly and sectioned into 300 µm thick slices using a vibroslicer (Leica). The slices were maintained at room temperature in a holding chamber containing oxygenated artificial cerebrospinal fluid (ACSF; 129 mM NaCl, 1 mM NaH2PO4, 10 mM Glucose, 1,8 mM MgS04, 1,6 mM CaCl2, 3 mM KCl, NaHC03 21 mM). (Ringer: 119 mM NaCl, 1 mM NaH2PO4, 10 mM Glucose, 1.3 mM MgSO4, 2.5 mM CaCl2, 2.5 mM KCl, NaHC03 26 mM) pH 7,2-7,4 Osmolarität 0,28-0,31 Osmol/kg
After at least 1 h equilibration, slices were transferred into submerged recording chamber and continuously perfuse with room temperature oxygenated ACSF at 3 ml/min throughout the experiments. For analyzing influence on LTP of Aβ1-42 wt and mutant peptide, slices were incubated with 500 nM peptide 20 min prior to tetanisation. Peptide was not washed during the experiment.

Field excitatory postsynaptic potentials (fEPSPs) were recorded of stratum radiatum area CA1 evoked by Schaffer collateral stimulation with ACSF filled electrodes. Two independent pathways were measured whereas two stimulating electrodes lay parallel to each other.

Baseline responses were collected using test pulses that yield 30- 40% of the maximal fEPSP slope. LTP was induced by Theta brust stimulation (TBS) of one of the two pathways. The inter-theta burst stimulation interval was 20 sec and four TBSs were applied to induce LTP. TBS consisted of 10 bursts delivered at 5 Hz. Each burst consisted of four pulses delivered at 100 Hz with a pulse-duration of 0.2 msec. (Evers M., Salmen B., Schachner M., 2002 J. Neuroscience)

After TBS, fEPSPs were observed for 30min. The amount of LTP was described as percent changes of fEPSP slopes after TBS normalized to baseline values with an additional subtraction of the second, unstimulated pathway. Slopes were analysed using IGOR program.

### Example 2

### Characterization of oligomers of Aβ1-42 by SEC

In this study it was investigated to what extent the GxxxG motif effects the aggregation of Aβ1-42 peptides. Aβ1-42 peptides and the oligomers were separated by use of SEC. As shown in Figure 1, the primarily formed species are 4- to 6mers of Aβ1-42. Interestingly, mutation of G₃₃ to A₃₃ or I₃₃ induces a shift to the formation of oligomers having a higher MW (16- to 20mers). For the G33A mutation minor amounts of tetramers are still detectable.

### Example 3

### Determination of the toxic effects of oligomers on human neuroblastoma cells

The toxic effects of various oligomers on human neuroblastoma cells was determined by use of the MTT test allowing to quantify the toxicity. The results are shown in Figure 2. Toxicity tests employing various SEC fractions show that Aβ1-42wt as well as mutations at position 29 induce 60% toxicity, whereas mutations at position 33 do not result in toxicity which is evident from the number of living cells as determined by the MTT assay. The study of particular SEC fractions clearly reveals that purified tetramers exhibit the highest toxicity (60%). Interestingly, after incubation of the cells with G33A mutant tetramers no toxic effects were observed.

Form these results it can be concluded that - in contrast to the accepted opinion - Aβ oligomers do not generally trigger loss of neurons and cognitive deficiencies but for doing so specific oligomers characterized by a particular structure must be present. This conclusion is based on the observation that peptides having a substituted glycine residue at position 33 are capable of forming oligomers which, however, are not toxic. For the Aβ wild type peptide it could be shown that basically tetramers cause toxicity resulting in the decease of neuroblastoma cells. The same results were observed with primary neurons (Figure 3).

### Example 4

### Interference effects of Aβ oligomers on learning and commemoration

In order to assay whether Aβ oligomers show an effect on learning and commemoration LTP (long term potentiation) of acute hippocampal sections of rat brains was determined.

Several recent publications report on the inhibition of LTP by Aβ1-42. This effect can be equated with cognitive deficiencies observed for AD patients. In order to further improve these performance tests and to minimize possible side effects of the peptides on the general network plasticity of the sections, two independent neuronal pathways were assayed. Whereas the first pathway was stimulated for inducing LTP, the second one was assayed unstimulated as a control. It could be confirmed that Aβ peptides inhibit LTP. However, in the presence of (a) Aβ1-42G33I or (b) tetramers of the Aβ1-42G33A peptide LTP could be induced and, as a consequence, "learning" was not adversely affected. These data indicate that toxicity directly correlates with the disturbance of "learning". Thus, in the present study it could be demonstrated for the first time that inhibition of LTP and toxicity are caused by the same molecules (i.e. wild type dimers and particularly tetramers). However, it became apparent that oligomers *per se* do not interfere with the "learning" but a particular specific structure of the oligomer is a prerequisite for inducing the effects described above.

In summary, the results of the present studies show that in particular wild type tetramers play a key role for inducing loss of neurons, are responsible for cognitive deficiencies and represent a target for new therapeutic interventions. In addition, it was shown that the negative effects can be blocked by a single substitution at position 33 (glycine).

## Claims

1. A method of screening for a therapeutic agent useful in the prophylaxis or treatment of Alzheimer's Disease (AD) comprising the steps of
(a) detecting oligomers of an Aβ peptide comprising an G₂₉xxxG₃₃ motif in an assay system under conditions allowing oligomerisation of said peptide and to which system a test compound has been added; and
(b) comparing the size distribution of the oligomers of said peptide and/or degree of oligomerization of Aβ with a control assay **characterized by** the absence of said test compound
wherein reduction of the amounts of toxic Aβ oligomers indicates that said test compound might be suitable for prophylaxis or treatment of AD.

2. The method of claim 1, wherein said Aβ oligomers are dimers or tetramers.

3. The method of claim 1 or 2, wherein said oligomers of the Aβ peptide contain a detectable label.

4. The method of claim 3, wherein the label is selected from the group consisting of a radioisotope, a bioluminescent compound, a chemiluminescent compound, a fluorescent compound, a metal chelate, or an enzyme.

5. The method of any one of claims 1 to 4, wherein the assay system is an in vitro system.

6. The method of any one of claims 1 to 4, wherein the assay system is a cell based system comprising cells which express a gene encoding said peptide.

7. The method of claim 6, wherein the cell based system is a neuronal cell culture.

8. The method of any one of claims 1 to 4, wherein the assay system is a non-human animal model.

9. The method of claim 8, wherein the non-human animal is a transgenic mouse.

10. The method of claim 8 or 9, wherein the non-human animal is transformed to express a gene encoding said peptide.

11. The method of any one of claims 1 to 10, wherein said Aβ peptide is Aβ40 or Aβ42 or a fragment thereof containing the APP-G₂₉xxxG₃₃ motif.

12. The method of any one of claims 1 to 11, wherein the APP-G₂₉xxxG₃₃ motif of said peptide is mutated.

13. The method of claim 12, wherein the G₃₃ of the APP-G₂₉xxxG₃₃ motif is mutated.

14. The method of claim 13, wherein G₃₃ has been replaced by A₃₃ or I₃₃.

15. The method of any one of claims 1 to 14, wherein the expected reduction of the amounts of dimers and/or tetramers is determined by use of a binding substance the binding affinity of which depends on the degree of oligomerization.

16. The method of claim 15, wherein said binding substance is an antibody.

17. The method of claim 16, wherein said antibody is a monoclonal antibody.

18. The method of any one of claims 1 to 14, wherein the expected reduction of the amounts of dimers and/or tetramers is determined by determination of the MW.

19. The method of claim 18, wherein the MW is determined by SEC (Size Exclusion Chromatography) or Western Blots.

20. The method of any one of claims 8 to 14, wherein the expected reduction of the amounts of dimers and/or tetramers is determined via the determination of toxicity.

21. The method of claim 20, wherein toxicity is determined by use of MTT and/or by monitoring behavioural alterations.

22. An antibody specifically binding to a dimer and/or tetramer of a peptide comprising an APP-G₂₉xxxG₃₃ motif for vaccination (a) of a subject suffering from AD or (b) for limiting or eliminating a risk to contract AD.

23. A dimer or tetramer of a peptide comprising an APP-G₂₉xxxG₃₃ motif.

24. The dimer or tetramer of claim 23, wherein said Aβ peptide is Aβ40 or Aβ42 or a fragment thereof containing the APP-G₂₉xxxG₃₃ motif.

25. A peptide comprising an APP-G₂₉xxxG₃₃ motif wherein the G₃₃ of the APP-G₂₉xxxG₃₃ motif is mutated.

26. The peptide of claim 26, wherein G₃₃ has been replaced by A₃₃ or I₃₃.
